**Europäisches Patentamt**

**European Patent Office**

**Office européen des brevets**

(19)

(11) Veröffentlichungsnummer: **0 087 678**
**B1**

(12)

# EUROPÄISCHE PATENTSCHRIFT

(45) Veröffentlichungstag der Patentschrift:
**18.12.85**

(21) Anmeldenummer: **83101452.7**

(22) Anmeldetag: **16.02.83**

(51) Int. Cl.⁴: **C 07 D 307/60, C 07 D 307/89**

(54) Verfahren zur kontinuierlichen Gewinnung von Phthalsäureanhydrid und Maleinsäureanhydrid aus Reaktionsgasen.

(30) Priorität: **27.02.82 DE 3207208**

(43) Veröffentlichungstag der Anmeldung:
**07.09.83 Patentblatt 83/36**

(45) Bekanntmachung des Hinweises auf die Patenterteilung:
**18.12.85 Patentblatt 85/51**

(84) Benannte Vertragsstaaten:
**AT BE DE IT**

(56) Entgegenhaltungen:
**DE - A - 1 056 128**
**DE - A - 2 444 824**

(73) Patentinhaber: **BASF Aktiengesellschaft,
Carl-Bosch-Strasse 38, D-6700 Ludwigshafen (DE)**

(72) Erfinder: **Danz, Eckehard, Dr.,
Hermann-Hesse-Strasse 42, D-6700 Ludwigshafen (DE)**
Erfinder: **Duembgen, Gerd, Dr., Sudetenstrasse 4,
D-6701 Dannstadt-Schauernheim (DE)**
Erfinder: **Miesen, Ernest, Samariterstrasse 3,
D-6700 Ludwigshafen (DE)**
Erfinder: **Schmidt, Joahnnes E., Dr., Pariser Strasse 23,
D-6700 Ludwigshafen (DE)**
Erfinder: **Wirth, Friedrich, Dr., Schlossgasse 6,
D-6700 Ludwigshafen (DE)**

## Beschreibung

Diese Erfindung betrifft ein neues kontinuierliches Verfahren zur Gewinnung von Phtalsäureanhydrid und Maleinsäureanhydrid aus Reaktionsgasen, die bei der katalytischen Luftoxidation von o-Xylol oder Naphtalin erhalten werden

Phthalsäureanhydrid wird grosstechnisch durch katalytische Luftoxidation von o-Xylol oder Naphthalin hergestellt. Man erhält dabei Reaktionsgase, die beispielsweise 80 g/Nm³ Phthalsäureanhydrid, 5 g/Nm³ Maleinsäureanhydrid und 60 g/Nm³ Wasserdampf enthalten, und die nach der Wärmerückgewinnung noch eine Temperatr von etwa 180° C aufweisen. Man scheidet das Phthalsäureanhydrid aus den Reaktionsgasen durch Desublimation an wärmeübertragenden Flächen ab. Dazu werden grosse Rippenrohrkondensatoren verwendet, an denen in der Beladungsphase durch Kühlen der Rohre das Phthalsäureanhydrid desublimiert, welches dann durch periodisches Aufheizen abgeschmolzen wird. Das rohe Phthalsäureanhydrid wird schliesslich in einer Reinigungsstufe, vorzugsweise durch Destillation von Nebenprodukten befreit. Da eine technische Anlage die Installierung mehrerer solcher Desublimatoren erfordert, entfällt auf die Abscheidung des Phthalsäureanhydrids aus den Reaktionsgasen ein hoher Aufwand an Investition, Reparatur und Energie. Ausserdem lässt das periodische Abkühlen und Aufheizen keine kontinuierliche Fahrweise zu.

Das in den Reaktionsgasen ebenfalls enthaltene Maleinsäureanhydrid scheidet sich nur partiell in den Desublimatoren ab. Für die Gewinnung des Maleinsäureanhydrids ist es deshalb erforderlich, die Reaktionsgase einer nachfolgenden Wasserwäsche zu unterziehen. Die dabei erhaltene wässrige Maleinsäurelösung muss dann in einem wiederum energieintensiven Verfahren eingedampft und zu Maleinsäureanhydrid dehydratisiert werden. Dafür werden hohe Temperaturen und teure korrosionsfeste Werkstoffe benötigt. Das Maleinsäureanhydrid wird danach noch destillativ gereinigt.

Um diese aufwendige Technik zu vereinfachen, hat man schon den Versuch gemacht, die Anhydride durch eine Wäsche der Reaktionsgase mit Lösungsmitteln zu isolieren. So wird in der US-PA Nr. 2942005 ein Verfahren beschrieben, bei dem man eine Wäsche mit Dibutyl- oder Dipropylphthalat als Lösungsmittel vornimmt. Das absorbierte Maleinsäureanhydrid wird abdestilliert und das Phthalsäureanhydrid durch Kühlen des Rückstandes auskristallisiert und mechanisch abgetrennt. Das Lösungsmittel wird zur Gaswäsche zurückgeführt, während das Phthalsäureanhydrid noch mehreren Reinigungsschritten unterworfen werden muss, um zuerst Lösungsmittelreste und anschliessend weitere Verunreinigungen zu entfernen.

Nach den Angaben der GB-PA Nr. 832619 wird das Reaktionsgas mit Kohlenwasserstoffen, die über 200° C sieden, wie Tetradecan oder Pentadecan, gewaschen. Nach einer destillativen Vorabtrennung des Maleinsäureanhydrids wird das Phthalsäureanhydrid durch Azeotropdestillation gewonnen. Die dabei entstehenden Azeotrope bestehen jedoch überwiegend aus den Kohlenwasserstoffen. Aus diesen Gemischen wird Phthalsäureanhydrid durch Phasentrennung abgeschieden. Es weist noch einen Gehalt an Kohlenwasserstoffen von 4 bis 5 Gew.% auf, der destillativ entfernt werden muss.

In der DE-A Nr. 2313306 wird eine Gaswäsche mit $C_{26-44}$-Paraffinen beschrieben. Das Phthalsäureanhydrid wird bei dieser Methode durch Kristallisation und mechanische Abtrennung aus der Maische isoliert. Das restliche Lösungsmittel muss jedoch wiederum destillativ abgetrennt werden.

Nach den Angaben der DE-A Nr. 2444824 kann man Maleinsäureanhydrid aus Prozessgasen mit solchen aromatischen Kohlenwasserstoffen auswaschen, die Molekulargewichte von 150 bis 400 und Siedepunkte von über 140° C aufweisen. Aus den dabei gewonnenen Lösungen wird das Maleinsäureanhydrid dann abdestilliert.

Bei dem in der DE-A Nr. 1056128 beschriebenen Verfahren werden die Anhydrid enthaltenden Prozessgase im Gegenstrom mit einem nicht mit Wasser mischbaren Lösungsmittel gewaschen. Bei der anschliessenden Aufarbeitung wird der Lösung des Anhydrids ein aliphatischer Kohlenwasserstoff mit einem Siedepunkt von über 190° C als Schleppmittel zugesetzt, dann wird das Gemisch destilliert und das Anhydrid aus dem Destillat durch Phasentrennung isoliert.

Diese bekannten Verfahren haben den gemeinsamen Nachteil, dass die Reinigung des Phthalsäureanhydrids vom verwendeten Lösungsmittel umständlich ist und besondere Massnahmen erfordert.

Schliesslich ist auch schon vorgeschlagen worden, die Reaktionsgase zur Gewinnung von Phthalsäureanhydrid und Maleinsäureanhydrid mit einem Gemisch dieser Anhydride zu behandeln (DE-A Nr. 2855629 und DE-A Nr. 2855630). Bei diesen mehrstufigen Absorptionsverfahren ist es zur vollständigen Gewinnung des Maleinsäureanhydrids erforderlich, das Abgas einer Wasserwäsche zu unterziehen. Man muss also auch hier die oben erwähnten Nachteile der Isolierung von Maleinsäureanhydrid aus wässrigen Maleinsäurelösungen in Kauf nehmen.

Es wurde nun gefunden, dass man bei der kontinuierlichen Gewinnung von Phthalsäureanhydrid und Maleinsäureanhydrid aus den Reaktionsgasen, die man bei der katalytischen Luftoxidation von o-Xylol oder Napthalin erhält, durch Behandlung der Reaktionsgase mit einem mit Wasser nicht mischbaren organischen Lösungsmittel bei erhöhter Temperatur im Gegenstrom und unter Verwendung eines zweiten organischen Lösungsmittels bei der Aufarbeitung die erwähnten Nachteile vermeidet, wenn man

a) die Reaktionsgase mit einer Temperatur von über 135° C in den unteren Bereich einer adiabatisch betriebenen Kolonne einleitet, als organisches mit Wasser nicht mischbares Lösungsmittel m-, o- oder p-Xylol, Ethylbenzol, n-Nonan oder iso-Nonan in den oberen Bereich der Kolonne eingibt, wobei man die Menge des Lösungsmittels so bemisst, dass weder nennenswerte Mengen des Lösungsmittels mit dem Sumpfprodukt abgezogen werden, noch nennenswerte Mengen der Anhydride mit dem Gas den Kopf der Kolonne verlassen,

b) die den Kopf der Kolonne verlassenden extrahierten Reaktionsgase zur Rückgewinnung von Resten des Lösungsmittels in einer Kolonne im Gegenstrom mit einem zweiten organischen Lösungsmittel, das im Temperaturbereich 180 bis 290° C siedet und mit dem ersten Lösungsmittel mischbar ist, behandelt und

c) das im wesentlichen aus Phthalsäureanhydrid und Maleinsäureanhydrid bestehende Sumpfprodukt aus der ersten Kolonne flüssig abzieht.

Nach dem neuen Verfahren erhält man die beiden Anhydride besonders vorteilhaft gemeinsam in Form eines flüssigen Gemisches, das von dem verwendeten Lösungsmittel weitgehend frei ist. Ausserdem wird eine nennenswerte Absorption des in den Reaktionsgasen enthaltenen Wasserdampfes vermieden.

Für das erfindungsgemässe Verfahren sind alle Reaktionsgase geeignet, die bei der bekannten katalytischen Luftoxidation von o-Xylol oder Naphthalin erhalten werden. Diese Reaktionsgase enthalten z.B. 35 bis 100 g/Nm³ Phthalsäureanhydrid und 1 bis 5 g/Nm³ Maleinsäureanhydrid.

Die Reaktionsgase werden nach dem Verfahren der Erfindung bei Temperaturen von über 135° C in den unteren Bereich einer adiabatisch betriebenen Kolonne eingeleitet. Beispielsweise wählt man Temperaturen von 135 bis 200° C, vorzugsweise 150 bis 180° C. Als Kolonnen eignen sich z.B. Bodenkolonnen mit einer Bodenzahl von 10 bis 20.

In den oberen Bereich der Kolonne leitet man das Lösungsmittel ein. Die erfindungsgemäss zu verwendenden Lösungsmittel haben bei dieser Arbeitsweise den grossen Vorteil, dass die Absorption der Anhydride aus dem Reaktionsgas, die in der Kolonne stattfindet, keine zusätzliche Energie erfordert. So befindet sich das Lösungsmittel auf den oberen Böden der Kolonne, während sich im Sumpf der Kolonne ein flüssiges Gemisch der Anhydride abscheidet, dessen Zusammensetzung dem Verhältnis der Komponenten im Reaktionsgas entspricht, wobei dieses flüssige Gemisch keine nennenswerten Mengen des Lösungsmittels enthält. Das flüssige Gemisch, das z.B. zu 94 Gew.% aus Phthalsäureanhydrid und zu 5 Gew.% aus Maleinsäureanhydrid besteht, wird dem Sumpf der Kolonne entnommen und auf an sich übliche Weise durch Destillation die reinen Anhydride aufgetrennt.

Obwohl bei diesem Verfahren relativ tiefe Temperaturen in der Absorptionskolonne auftreten und in Gegenwart von Wasserdampf gearbeitet wird, werden erstaunlicherweise keine Feststoffausscheidungen an Phthalsäureanhydrid, Phthalsäure oder Maleinsäure beobachtet, so dass ein vollkontinuierlicher Betrieb möglich ist.

Die den Kopf der Kolonne verlassenden Reaktionsgase, die mit dem Lösungsmittel gesättigt sind, werden zur Rückgewinnung des Lösungsmittels in einer zweiten Kolonne mit dem zweiten Lösungsmittel behandelt. Nach einer besonders zweckmässigen Ausführungsform der Erfindung werden die Reaktionsgase nach dem Verlassen der ersten Kolonne abgekühlt. Je nach den gegebenen technischen Möglichkeiten kühlt man auf Temperaturen von 40 bis 20° C ab. Dabei kondensiert ein grosser Teil des in den Reaktionsgasen enthaltenen organischen Lösungsmittels sowie des Reaktionswassers. Man kühlt die Gase zweckmässig in einem Quenchraum ab. Über ein Phasentrenngefäss wird das Wasser ausgeschleust. Das Lösungsmittel gibt man in geeigneter Menge als Rücklauf auf den obersten Boden der Absorptionskolonne. Da die Reaktionsgase nach der Abkühlung noch beträchliche Mengen an Lösungsmittel und Wasserdampf enthalten, werden die Gase zur Rückgewinnung des Lösungsmittels mit einem organischen Lösungsmittel, das im Temperaturbereich zwischen 180 und 290° C, vorzugsweise zwischen 220 und 240° C siedet, und das mit dem zu absorbierenden Lösungsmittel mischbar ist, im Gegenstrom behandelt. Diese Behandlung wird zweckmässig in einer Bodenkolonne mit 10 bis 20 Böden vorgenommen. Als Lösungsmittel der genannten Art kommen z.B. Kohlenwasserstoffe wie Diphenyl, aromatische Ether, wie Diphenyloxid oder Carbonsäuren, wie Iononansäuren oder Ethylhexansäure in Betracht. Besonders gut eignen sich Carbonsäuren, wie Ethylhexansäure, das z.B. bei der Verwendung von o-Xylol als Lösungsmittel und einer Kolonne mit 10 bis 20 Böden eine nahezu quantitative Absorption ermöglicht. Bei der Absorption mit Hilfe des hochsiedenden Lösungsmittels werden auch noch geringe Mengen an Wasser absorbiert. Die im Sumpf der Absorptionskolonne anfallende Wasser enthaltende Mischung der beiden Lösungsmittel wird zweckmässigerweise in einer Destillationskolonne getrennt.

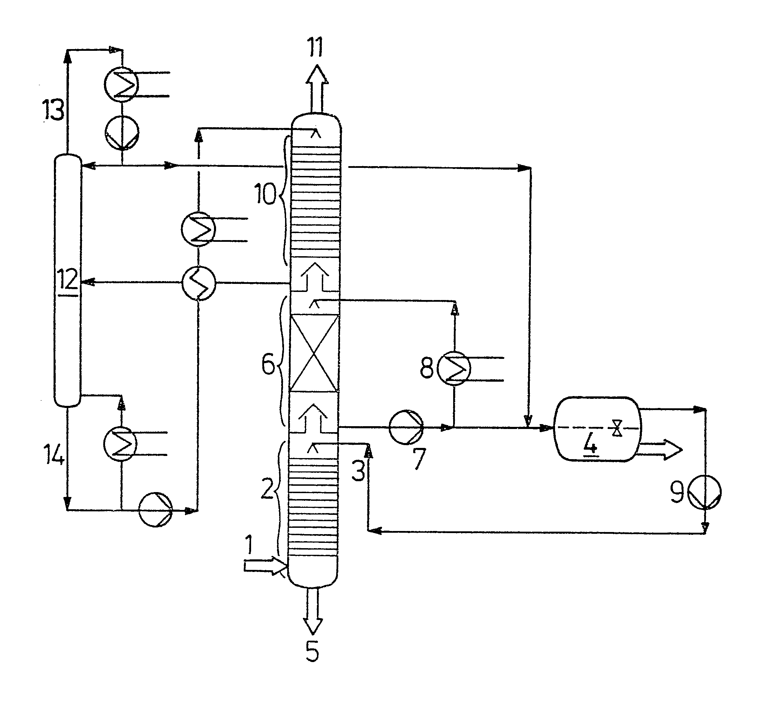

Das Kopfprodukt der Destillationskolonne, das das leichter flüchtige Lösungsmittel und Wasser enthält, wird in das oben erwähnte Trenngefäss gegeben. Im Sumpf der Destillationskolonne, die gegebenenfalls unter einem entsprechenden Vakuum betrieben werden kann, fällt das hochsiedende organische Lösungsmittel an, das zweckmässigerweise auf den Kopf der Absorptionskolonne geleitet wird. Geringe verschmutzte Mengen können bei Bedarf ausgeschleust und ergänzt werden. Das erfindungsgemässe Verfahren wird an Hand der Figur und dem nachfolgenden Beispiel näher erläutert.

*Beispiel*

Das durch katalytische Luftoxidation von o-Xylol erhaltene Prozessgas, das eine Temperatur von 180° C aufweist und mit 80 g/Nm³ Phthalsäureanhydrid, 5 g/Nm³ Maleinsäureanhydrid und 60 g/Nm³ Wasserdampf beladen ist, wird in der durch die Figur erläuterte Weise von unten (1) in eine adiabatisch betriebene Kolonne mit 17 Böden (2) eingeleitet. Auf den Kopf der Kolonne (3) wird o-Xylol mit 30° C aus dem Trenngefäss (4) gegeben. Die Menge ist so einreguliert, dass weder nennenswerte Mengen o-Xylol den Sumpf der Kolonne (2) verlassen, noch Phthalsäureanhydrid oder Maleinsäureanhydrid in nennenswerten Mengen mit dem Gas aus der Kolonne ausgetragen werden. In der adiabatisch betriebenen Kolonne stellt sich ein Temperaturprofil ein, das zur Regelung der Menge an o-Xylol herangezogen wird.

Es ergeben sich Temperaturen von 142° C im Sumpf, abfallend auf 70° C auf dem obersten 17. Boden. Eine Kondensation des Wasserdampfes tritt dabei noch nicht auf (Taupunkt ca. 40° C). Die flüssige Mischung aus Phthalsäureanhydrid und Maleinsäureanhydrid verlässt den Sumpf der Kolonne (5) mit einem Restgehalt von unter 0,01 Gew.% o-Xylol und ca. 0,5 Gew.% der jeweiligen Säuren. Sie wird anschliessend destillativ getrennt. Das vom Kopf der Kolonne (2) in den Quenchraum (6) übertretende Gas wird auf 30° C abgekühlt. Zu dem Quench gehören eine Pumpe (7) und ein mit Kühlwasser betriebener Wärmetauscher (8). Das im Quench (6) anfallende flüssige Gemisch aus o-Xylol und Wasser wird im Trenngefäss (4) getrennt. Die obere Phase aus o-Xylol geht über eine Pumpe (9) zurück auf den Kopf der Kolonne (3). Es werden pro Nm³ Reaktionsgas 0,43 kg o-Xylol benötigt. Das Wasser aus dem Trenngefäss (4), das gegebenenfalls noch durch Strippung von Resten o-Xylol befreit werden kann, enthält nur geringe Mengen Phthalsäure und Maleinsäure als Verlust aus der Kolonne (2). Die Gesamtmenge beträgt etwa 0,1% der Ausbeute an Phthalsäure- und Maleinsäureanhydrid zusammen gerechnet. Nach dem Quenchraum (6) durchläuft das Gas bei 30° C die aufgesetzte Absorptionskolonne (10), in welcher das Gas zur Absorption von Resten an Lösungsmittel mit Ethylhexansäure behandelt wird. Diese Kolonne besitzt 15 Böden, was bei einer Waschflüssigkeitsmenge an Ethylhexansäure von 0,24 kg/Nm³ Gas ausreicht, um am Kopf der Absorptionskolonne Verluste an o-Xylol von nur 0,05 g/Nm³ zu erreichen. Das Abgas (11) enthält ferner bei 30° C noch eine Restmenge an Ethylhexansäure von 0,1 g/Nm³. Im Sumpf der Absorptionskolonne (10) fällt eine Mischung aus 80,4 Gew.% Ethylhexansäure, 19,0 Gew.% o-Xylol und 0,6 Gew.% Wasser an. Dieses Gemisch wird zur Destillationskolonne (12) geleitet, in der als Kopfprodukt (13) o-Xylol und Wasser erhalten werden, die in das Trenngefäss (4) geleitet werden. Die Ethylhexansäure wird als Sumpfprodukt (14) nach einer Kühlung auf 30° C zurück zum Kopf der Absorberkolonne (10) geleitet. Die Destillation (12) kann unter Normaldruck betrieben werden, vorzugsweise jedoch wird mit einem Vakuum von 130 mbar gearbeitet. Ein Teilstrom der Ethylhexansäure (2 bis 3%) wird ausgeschleust und destillativ gereinigt.

## Patentansprüche

1. Verfahren zur kontinuierlichen Gewinnung von Phthalsäureanhydrid und Maleinsäureanhydrid aus den Reaktionsgasen, die man bei der katalytischen Luftoxidation von o-Xylol oder Naphthalin erhält, durch Behandlung der Reaktionsgase mit einem mit Wasser nicht mischbaren organischen Lösungsmittel bei erhöhter Temperatur im Gegenstrom und unter Verwendung eines zweiten organischen Lösungsmittels bei der Aufarbeitung, dadurch gekennzeichnet, dass man

a) die Reaktionsgase mit einer Temperatur von über 135° C in den unteren Bereich einer adiabatisch betriebenen Kolonne einleitet, als organisches mit Wasser nicht mischbares Lösungsmittel m-, o- oder p-Xylol, Ethylbenzol, n-Nonan oder iso-Nonan in den oberen Bereich der Kolonne eingibt, wobei man die Menge des Lösungsmittels so bemisst, dass weder nennenswerte Mengen des Lösungsmittels mit dem Sumpfprodukt abgezogen werden, noch nennenswerte Mengen der Anhydride mit dem Gas den Kopf der Kolonne verlassen,

b) die den Kopf der Kolonne verlassenden extrahierten Reaktionsgase zur Rückgewinnung von Resten des Lösungsmittels in einer Kolonne im Gegenstrom mit einem zweiten organischen Lösungsmittel, das im Temperaturbereich 180 bis 290° C siedet und mit dem ersten Lösungsmittel mischbar ist, behandelt und

c) das im wesentlichen aus Phthalsäureanhydrid und Maleinsäureanhydrid bestehende Sumpfprodukt aus der ersten Kolonne flüssig abzieht.

2. Verfahren nach Anspruch 1, dadurch gekennzeichnet, dass man als Lösungsmittel für Stufe a) o-Xylol verwendet.

3. Verfahren nach Anspruch 1, dadurch gekennzeichnet, dass man als zweites organisches Lösungsmittel des Siedebereichs 180 bis 290° C Ethylhexansäure verwendet.

4. Verfahren nach Anspruch 1, dadurch gekennzeichnet, dass man die den Kopf der ersten Kolonne verlassenden Reaktionsgase auf eine Temperatur von 40 bis 20° C abkühlt.

## Claims

1. A process for the continuous isolation of phthalic anhydride and maleic anhydride from the reaction gases obtained by catalytic oxidation of o-xylene or naphtalene with air, by treating the reaction gases countercurrently with a water-immiscible organic solvent at elevated temperature, and using a second organic solvent in the working-up step, wherein

(a) the reaction gases, at a temperature of more than 135° C, are introduced into the lower zone of a column, and m-, o- or p-xylene, ethylbenzene, n-nonane or isononane, as the water-immiscible organic solvent, is introduced into the upper zone of the column, the amount of solvent being such that neither appreciable amounts of solvent are discharged with the bottoms product, nor appreciable amounts of the anhydrides leave the top of the column with the gas,

(b) to recover residual solvent, the extracted reaction gases leaving the top of the column are treated countercurrently in a column with a second organic solvent which has a boiling range of from 180 to 290° C and is miscible with the first solvent, and

(c) the bottoms product form the first column, which consists essentially of phthalic anhydride and maleic anhydride, is taken off as liquid.

2. A process as claimed in Claim 1, wherein o-xylene is used as the solvent for step (a).

3. A process as claimed in Claim 1, wherein ethylhexanoic acid is used as the second organic solvent with a boiling range of from 180 to 290° C.

4. A process as claimed in Claim 1, wherein the reaction gases which leave the top of the first column are cooled to from 40 to 20° C.

**Revendications**

1. Procédé pour l'extraction en continu de l'anhydride phtalique et de l'anhydride maléique de gaz de réaction, produits lors de l'oxydation par l'air catalysée de l'ortho-xylène ou du napthalène, par traitement des gaz réactionnels à température accrue et à contre-courant avec un solvant organique non miscible à l'eau, un deuxième solvant organique étant employé dans une opération subséquente, caractérisé en ce que:

a) l'on introduit les gaz réactionnels à une température supérieure à 135° C dans la partie inférieure d'une colonne à fonctionnement adiabatique, dans la partie supérieure de laquelle on introduit un solvant organique non miscible à l'eau, choisi parmi le m-, le o- et le p-xylène, l'éthylbenzène, le n-nonane et l'iso-nonane, en une quantité telle qu'il n'y ait ni soutirage de proportions notables du solvant avec le produit résiduel, ni évacuation de quantités notables d'anhydrides avec le mélange gazeux au sommet de la colonne;

b) l'on traite les gaz réactionnels extraits, quittant la colonne par le sommet, dans une deuxième colonne, en contre-courant, avec un deuxième solvant organique, possédant un point d'ébullition dans la gamme de 180 à 290° C et miscible avec le premier solvant, en vue de l'extraction du (premier) solvant résiduel;

c) l'on soutire de la première colonne, à l'état liquide, un produit résiduel constitué essentiellement d'anhydride phtalique et d'anhydride maléique.

2. Procédé suivant la revendication 1, caractérisé en ce que, dans le stade a), on emploie comme solvant de l'ortho-xylène.

3. Procédé suivant la revendication 1, caractérisé en ce que le deuxième solvant organique, bouillant entre 180 et 290° C, est l'acide éthylhexanoïque.

4. Procédé suivant la revendication 1, caractérisé en ce que les gaz réactionnels, quittant le sommet de la première colonne, sont refroidis entre 40 et 20° C.